# EUROPÄISCHE PATENTANMELDUNG

(11) **EP 2 474 285 A1**
(43) Veröffentlichungstag der Anmeldung: **11.07.2012**
(21) Anmeldenummer: 12000024.5
(22) Anmeldetag: 03.01.2012
(51) Int. Cl.: A61B 19/02, A61B 13/00

(54) **Vorrichtung zur Aufbewahrung von medizinischen Spateln**

(30) Priorität: 06.01.2011 DE 202011001140 U
(71) Anmelder: Gaede, Robin, 48291 Telgte (DE)
(72) Erfinder: Gaede, Robin, 48291 Telgte (DE)
(74) Vertreter: Hoffmeister, Helmut

(57) **Zusammenfassung**

Die Aufbewahrung medizinischer Spatel erfolgt beispielsweise in Arztpraxen häufig lose in sehr einfachen Gefäßen. Um den Hygieneanforderungen besser zu genügen und Verschmutzungen durch Staub und insbesondere auch durch Keime zu reduzieren wird eine Vorrichtung zur Aufbewahrung und Abgabe einheitlich geformter medizinischer Spatel (2) vorgeschlagen, welches ein Gehäuse (1.1; 1.2) mit einem in Form eines quaderförmigen Hohlraums ausgestalteten Lagerraum (9) und einem Boden (5), in dem die Spatel auf dem Boden (5) des Gehäuses (1.1; 1.2) stapelbar sind, wobei das Gehäuse (1.1; 1.2) an seinem Fußbereich unmittelbar oberhalb des Bodens (5) mit einer Entnahmeöffnung (15) für einen einzelnen Spatel (2) versehen ist und bei dem die Öffnungslänge (L1) der größer ist als die Spatellänge (L2) sowie die Öffnungshöhe (H1) größer ist als die Spatelhöhe (H2) und wobei der Boden (5) wenigstens eine Zugriffsöffnung (6; 26) aufweist.

## Beschreibung

Die Erfindung betrifft eine Vorrichtung zur Aufbewahrung von medizinischen Spateln gemäß dem Oberbegriff des Patentanspruch 1.

Medizinische Spatel sind insbesondere Mundspatel, auch Abstrichspatel genannt. Es handelt sich meist um Blättchen aus Holz, mit welchen die Zunge eines Patienten nach unten gedrückt wird, um besser in den Hals-Rachenraum blicken zu können. Die Standardlänge beträgt 15 cm; auch andere Längen, wie 10 und 18 cm, sind in Gebrauch. Neben Holzspateln sind auch Plastik-Ausführungen bekannt.

Neben dem Einsatz für medizinische Zwecke finden ähnliche Spatel auch in anderen Bereichen Verwendung, beispielsweise zum Auftragen von Chemikalien beim Friseur, als Schiene in der Tiermedizin oder zum Basteln.

Die Aufbewahrung der Spatel erfolgt in Arztpraxen oder medizinischen Behandlungsräumen häufig lose in sehr einfachen Gefäßen, wie beispielsweise Gläsern oder Dosen. Zum Einsatz kommen in aller Regel nicht steril verpackte Einzelspatel, sondern offene Spatel, die in Gebinden von z.B. 100 Stück geliefert werden und zum bedarfweisen Nachfüllen der einfachen Vorratsbehältnisse dienen. Bis zur bestimmungsmäßigen Verwendung sind die Spatel den äußeren Umwelt-Einflüssen ausgesetzt. Negative Auswirkungen in Form von Verschmutzungen ergeben sich nicht nur durch Staub und sonstige in der Luft enthaltene Partikel, sondern insbesondere auch durch Keime, die, gerade auch in Arztpraxen, von erkrankten Patienten beispielsweise über die Atemluft, insbesondere durch Husten, verbreitet werden. Auch greifen Kinder bei Arztbesuchen oftmals zu den Spateln, weil sie sie für Spielzeug halten.

Aus CN 2296188 ist eine Aufbewahrungsbox bekannt, welche ein innerhalb der Box befindliches und mit Spateln befülltes Magazin enthält. Die Entnahme einzelner Spatel erfolgt mittels einer Förderschiene, welche zur Seite hin durch einen Schlitz transportiert wird, der so dimensioniert ist, dass ein einzelner Spatel auf der Schiene liegend durch den Schlitz gezogen werden kann, der darüber liegende Spatel jedoch von der oberen Begrenzung des Schlitzes zurückgehalten wird. Die Rückstellung des Schlittens erfolgt mittels eines den Schlitten mit einer Seitenwand verbindenden Federelementes. Nachteilig an dieser Ausführungsform ist das Erfordernis beweglicher, und damit in der Regel aufwendiger und störanfälliger Einzelkomponenten. Weiterhin nachteilig ist, dass nur eine seitliche Ausgabe der Spatel vorgesehen ist.

Der Erfindung liegt die Aufgabe zugrunde, eine Vorrichtung zur Aufbewahrung und Abgabe einheitlich geformter medizinischer Spatel zur Verfügung zu stellen, welche einerseits eine Kontamination der Spatel mit Schmutz und Krankheitserregern weitgehend reduziert und andererseits auf einfache und kostengünstige Art eine Einzelentnahme der Spatel in Quer- und/oder Längsrichtung, bezogen auf die Lage der Spatel in der Aufbewahrungsbox, durch eine befugte Person ermöglicht.

Diese Aufgabe wird gelöst, indem unter Verzicht auf den Einsatz störanfälliger mechanischer Komponenten eine geschlossene Aufbewahrungsvorrichtung der eingangs genannten Art mit einer Entnahmeöffnung versehen wird, welche, bezogen auf die Länge und Höhe eines Spatels, mit geringem, allseitigen Überstand versehen ist und bei der der Boden eine Zugriffsöffnung aufweist.

In einer vorteilhaften Ausführung befindet sich die Zugriffsöffnung im Mittelbereich des Bodens. Hierdurch wird die Gefahr, dass ein Spatel sich beim Herausnehmen verklemmt oder verkantet, reduziert.

Auch an einer Außenseite des Bodens kann die Zugriffsöffnung in den Boden eingearbeitet sein, d.h. durch eine Aussparung des Bodens gebildet sein. Bei dieser Ausführungsform wird der zuunterst liegende Spatel von der Unterseite her kontaktiert und wie eine Art Zeiger aus der Entnahmeöffnung mit dem Finger herausgeholt. Ein Kontakt erfolgt daher beim Herausziehen an einem Ende; die Hauptlänge des Spatels bleibt unberührt. Hierbei wird - für Rechtshänder - vorzugsweise die rechte Außenseite für die Position der Zugriffsöffnung gewählt.

Es ist auch möglich, beide Außenseiten mit einer Zugriffsöffnung zu versehen, beispielsweise, um Links- und Rechtshänder die Entnahme zu erleichtern. Schließlich kann in Kombination mit einem oder zwei außen liegenden Zugriffsöffnungen zusätzlich eine im Mittelbereich des Bodens liegen.

Um einerseits eine großflächige Auflagefläche für die Spatel auf dem Boden der Aufbewahrungsbox zur realisieren und andererseits dem Anwender eine bequeme Zugriffsmöglichkeit anzubieten, hat die Zugriffsöffnung vorzugsweise eine Größe, die das Einführen einer Fingerspitze erlaubt.

In einer ergonomisch günstigen Gestaltung weist die Zugriffsöffnung eine divergierende, abgerundete Kontur auf.

Als vorteilhaft hat sich erwiesen, zumindest eine, beziehungsweise die an der Außenseite befindliche, Zugriffsöffnung durch wenigstens eine in der Vorderseite des Gehäuses eingearbeitete Aussparung zu vergrößern.

In einer bevorzugten Ausführungsform ist die Rückwand des Spatelgehäuses größer als der Lagerraum. In dem den Spatel-Lagerraum überragenden Bereich der Rückwand sind Löcher vorgesehen, mit Hilfe derer der Vorratsbehälter direkt an einer Wand oder auf einem Tragegestell befestigt werden kann. Mit Hilfe des Tragegestells wird ein standsicheres Aufstellen beispielweise auf einem Schreibtisch, ermöglicht. Natürlich ist es auch denkbar, an Stelle einer Schraubverbindung einen Klebebereich zum Anbringen des Gehäuses an das Tragegestell vorzusehen oder andere, übliche Gestaltungsformen zu realisieren, die ein Aufhängen oder Aufstellen des Spatelgehäuses ermöglichen.

Um den Nutzer über den Spatel-Füllstand zu informieren, empfiehlt es sich, das Spatelgehäuse zumindest im Bereich der Entnahmeöffnung durchsichtig zu gestalten.

Für das Wiederauffüllen eines weitgehend geleerten Spatelgehäuses ist es günstig, wenn entweder die Oberseite oder eine Schmalseite mit einer Einfüllöffnung versehen ist.

In einer vorteilhaften Ausgestaltung ist diese Einfüllöffnung verschließbar, wobei als Verschlusselement beispielsweise eine Schieberzunge oder eine Klappe vorgesehen sein kann.

Soweit es sich um einen Ausführung mit an der Schmalseite befindlicher Einfüllöffnung handelt, wird die Stabilität des Gehäuses verbessert, indem ein Teil der Schmalseite oberhalb des Bodens mit einer Schwelle abgedeckt ist, die auch das Herausfallen von Spateln beim Aufschieben des Verschlusses verhindert.

Zumindest das Spatelgehäuse besteht vorzugsweise aus Kunststoff.

Ausführungsbeispiele der Erfindung werden anhand der beigefügten Zeichnung erläutert. Die Figuren der Zeichnung zeigen:
- Fig. 1: Ein Spatelgehäuse in perspektivischer Ansicht mit einer durch einen Schieber verschließbaren Einfüllöffnung an einer Schmalseite;
- Fig. 2: das Spatelgehäuse aus Fig. 1 in Vorderansicht;
- Fig. 3: das Spatelgehäuse aus Fig. 1 in Seitenansicht;
- Fig. 4: das Spatelgehäuse aus Fig. 1 in Draufsicht;
- Fig. 5: einen Spatel in perspektivischer Ansicht;
- Fig. 6: ein Spatelgehäuse, schräg von unten gesehen;
- Fig. 7: ein auf ein Gestell montiertes Spatelgehäuse mit einer durch eine Klappe verschließbaren Einfüllöffnung an der Oberseite:
- Fig. 8: ein Spatelgehäuse, in einer Ausführungsform mit zusätzlicher, seitlich angeordneter Entnahmeöffnung.

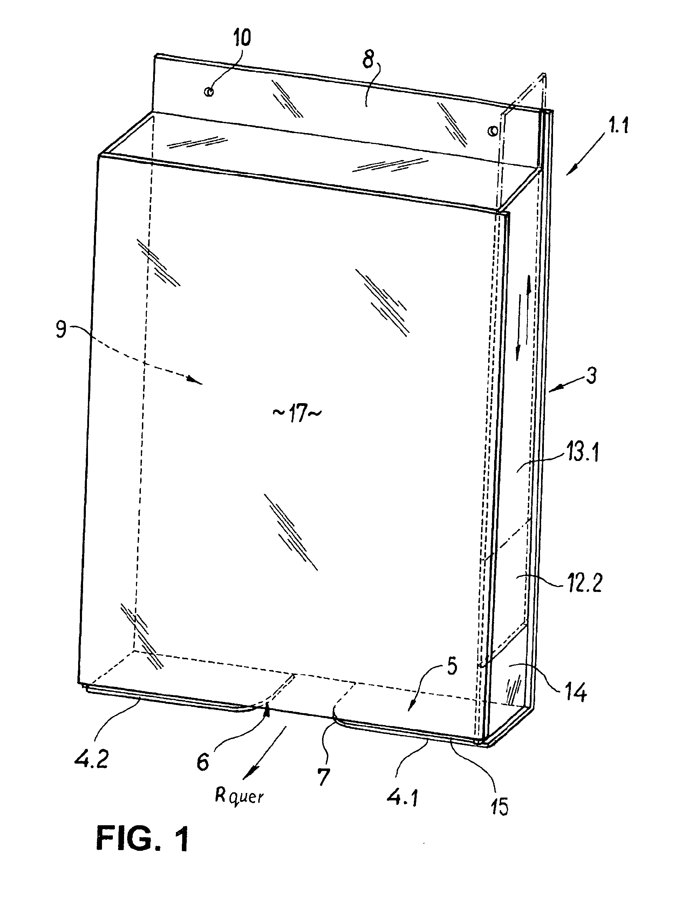

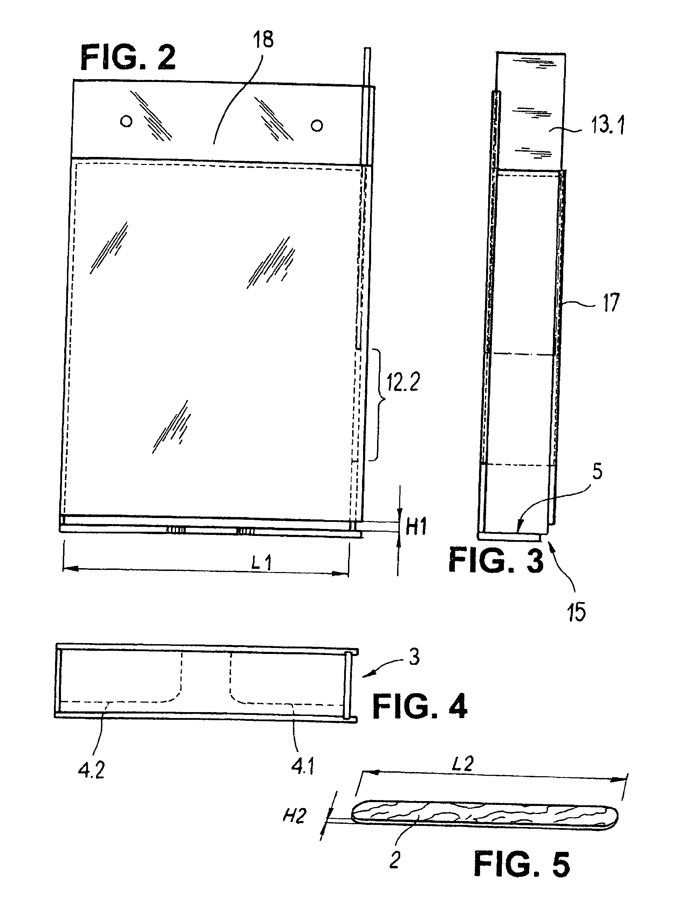

Die Figuren 1 bis 4 zeigen ein Spatelgehäuse, im folgenden Gehäuse 1.1 genannt, mit einer an der Schmalseite 3 befindlichen Einfüllöffnung 12.2 und mit einer Schieberzunge 13.1 in einer teilweise geöffneten Position (gestrichelt dargestellt). Der in Form eines quaderförmigen Hohlraums ausgestaltete Lagerraum 9 des Gehäuses 1.1 ist zur Aufnahme von Spateln 2 geeignet. Eine übliche Ausführungsform eines Spatels 2 ist in Figur 5 dargestellt.

Eine Anzahl von Spateln 2 ist auf dem Boden 5 des Gehäuses 1.1 gestapelt lagerbar. Die im Lagerraum 9 befindliche Fläche des Bodens 5 ist geringfügig größer als die Grundfläche eines Spatels 2. Das allseitige Übermaß des Lagerraums 9 ist so bemessen, dass einerseits ein Verklemmen der Spatel, welches bei zu geringem Übermaß eintreten könnte, verhindert wird und andererseits ein im Gehäuse 1.1 befindlicher SpatelStapel nicht auseinanderrutschen kann.

An einer Schmalseite 3 des Gehäuses 1.1 ist die Einfüllöffnung 12.2 angeordnet, durch die hindurch der Lagerraum 9 bei Bedarf mit Spateln 2 nachbefüllt werden kann. Die Einfüllöffnung 12.2 ist geringfügig breiter als die Spatelbreite, so dass die Spatel 2 stapelweise durch die Einfüllöffnung 12.2 in den Lagerraum 9 geschoben werden können.

Nach dem Befüllen des Lagerraums 9 kann die Einfüllöffnung 12.2 mit einer Schieberzunge 13.1 verschlossen werden. Um die Stabilität des Gehäuses 1.1 zu erhöhen und/oder einen Anschlag für die Schieberzunge 13.1 auszubilden, ist unterhalb der Einfüllöffnung 12.2 eine Schwelle 14 vorgesehen.

Der Boden 5 des Gehäuses 1.1 wird durch zwei Bodenplatten 4.1 und 4.2 gebildet, welche außenseitig zur Mitte des Gehäuses hin mit einer abgerundeten Kontur 7 versehen sind und zwischen sich eine Zugriffsöffnung 6 belassen, die mit einer Breite von ca. 25 mm etwas breiter ist als ein menschlicher Finger.

Die Vorderseite 17 mit der Unterkante 17.1 des Gehäuses 1.1 ist so zum Gehäuse positioniert, dass eine schlitzartige Entnahmeöffnung 15 direkt oberhalb des Bodens 5 verbleibt, welche sich über die Gesamtbreite des Bodens erstreckt. Diese ist hinsichtlich ihrer Öffnungshöhe H1 größer als die Höhe eines Spatels H2, und kleiner als die doppelte Spatelhöhe H2. Es ist somit nur möglich, den untersten Spatel des Stapels durch die Entnahmeöffnung 15 zu ziehen. Die verhältnismäßig große Freifläche der Vorderwand eignet sich daher gut dafür, mit einer Bedruckung oder Beklebung, z.B. in Form von Werbung, versehen zu werden. Ein Teil der Vorderseite ihm Bereich der Einfüllöffnung kann auch durchsichtig sein, so dass der Füllzustand kontrolliert werden kann.

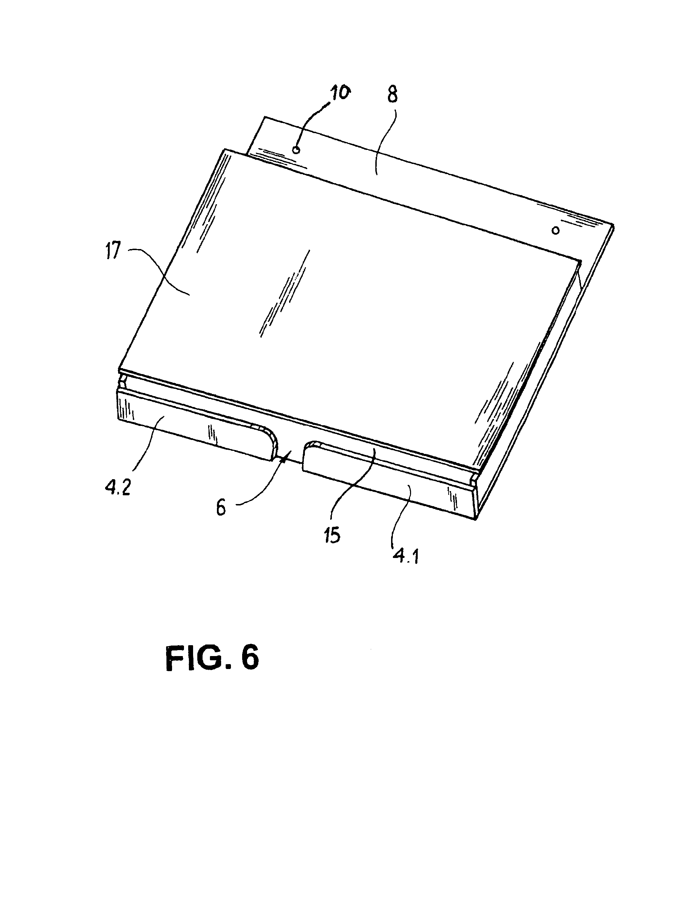

Figur 6 verdeutlicht die Gestaltung des Bodenbereiches bei einem Gehäuse in einer perspektivischen Darstellung. Die verbleibende Entnahmeöffnung 15 zwischen der Vorderseite-Unterkante 17.1 und Bodenplatten 4.1 und 4.2 ist deutlich zu erkennen, ebenso die Zugriffsöffnung 6.

Die Rückwand 8 des Gehäuses 1.1 überragt den Lagerraum 9 und weist zwei Löcher 10 auf, über die eine Schraubverbindung direkt an einer nicht dargestellten Wandfläche oder an einer Standvorrichtung 16 ermöglicht ist.

Die Entnahme eines Spatels 2 aus dem im Lagerraum 9 befindlichen Spatelstapel erfolgt, indem ein Finger im Bereich der Zugriffsöffnung 6 an den im Stapel unten liegenden Spatel herangeführt wird, diesen berührt und unter Beibehaltung der Berührung in Richtung der Entnahmeöffnung 15 zieht. Der leicht am Finger haftende Spatel gleitet durch die Entnahmeöffnung 15 und kann, soweit er das Gehäuse 1.1 zumindest teilweise verlassen hat, mit Hilfe eines Daumens gegriffen werden. Während des Entnahmevorgangs wird der zweitunterste Spatel durch die Unterkante der Vorderseite 17 zurückgehalten und legt sich, nachdem der unterste Spatel entnommen ist, auf dem Boden 5 ab. In analoger Form können weitere Spatel entnommen werden.

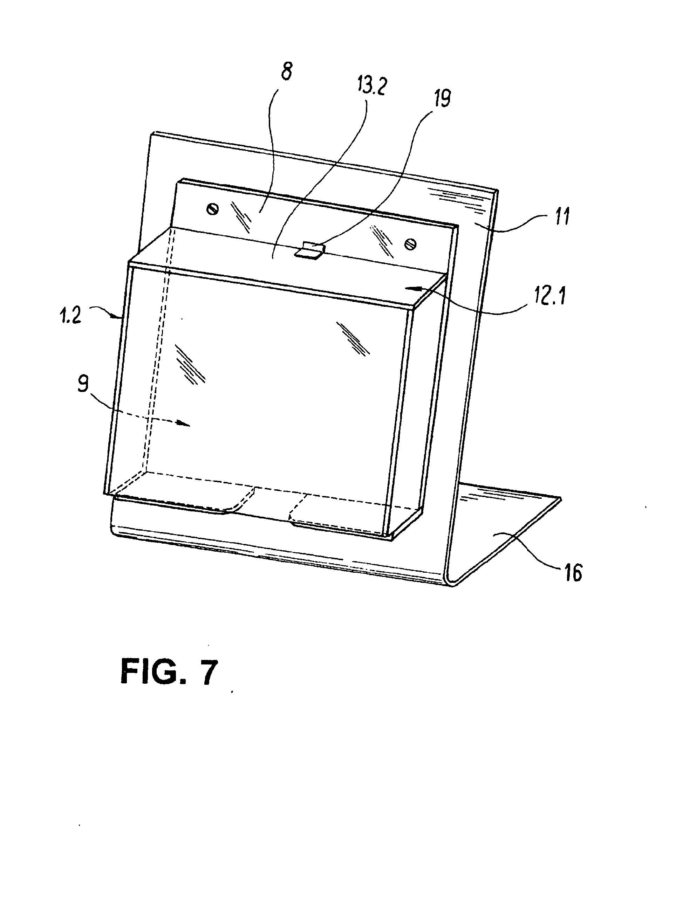

Figur 7 zeigt ein an einer Standvorrichtung 16 montiertes Gehäuse 1.2. Unabhängig davon, ob ein Gehäuse an eine Standvorrichtung montiert ist oder an einer Wand hängend, kann als Verschlusselement für die Einfüllöffnung 12.1 auch eine Klappe 13.2 vorgesehen sein. Die Klappe 13.2 ist mit einem geeigneten Element, beispielsweise einem Scharnier 19, mit der Rückwand 8 des Gehäuses verbunden.

Selbstverständlich ist es auch möglich, ein an einer Wand hängendes Gehäuse mit einer die Oberseite des Lagerraums 9 bedeckenden Klappe 13.2 auszuführen oder ein an einer Standvorrichtung 16 montiertes Gehäuse mit einer Schieberzunge 13.1 zu versehen.

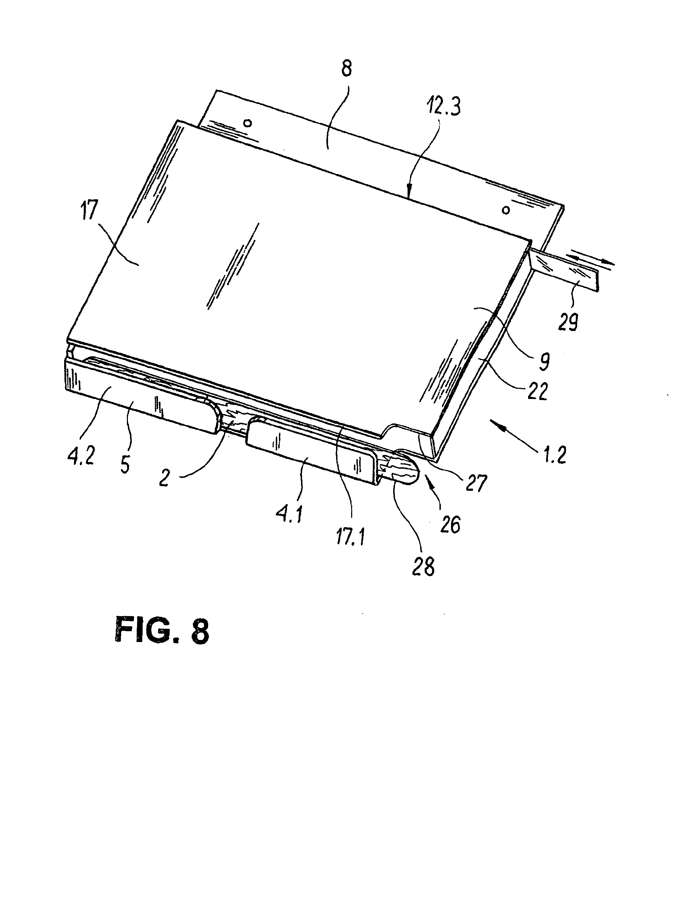

Figur 8 zeigt eine Ausführungsform eines Spatelgehäuses 1.2, bei dem der Boden 5 mit einer zusätzlichen seitlichen Zugriffsöffnung 26 auf der rechten Seite versehen ist (Figur 8). Diese Seite ist mit einer festen Seitenwand 22 ausgestattet. In die Zugriffsöffnung 26 ragt der Endbereich 28 eines Spatels 2 hinein. Die Zugriffsöffnung 26 hat etwa eine offene Länge von 15 bis 25 mm, so dass die Fingerspitze eines Mittelfingers Eingriff hat und die Unterseite des untersten Spatels somit kontaktiert werden kann. Die Vorderseite 17 des Gehäuses und die feste Seitenwand 22 können mit einer eingearbeiteten Aussparung 27 vergrößert sein. Auf die im Mittelbereich angeordnete Zugriffsöffnung kann damit verzichtet werden.

Um sowohl Rechts- als auch Linkshänder die Entnahme eines Spatels zu erleichtern, kann an der gegenüberliegenden linken Seite eine weitere Zugriffsöffnung spiegelbildlich zu der auf der rechten Seite vorgesehen sein.

Als Verschlusselement umfasst die obere Öffnung 12.3. des Lageraums 9 einen Verschluss in Form einer verstellbaren Schiebezunge 29.

### Bezugszeichenliste

| | |
|---|---|
| 1.1; 1.2 | Gehäuse |
| 2 | Spatel |
| 3 | Schmalseite |
| 4.1; 4.2 | Bodenplatte |
| 5 | Boden |
| 6 | Zugriffsöffnung |
| 7 | abgerundete Kontur |
| 8 | Rückwand |
| 9 | Lagerraum |
| 10 | Loch |
| 11 | Platte |
| 12.1; 12.2; 12.3 | Einfüllöffnung |
| 13.1 | Schieberzunge |
| 13.2 | Klappe |
| 14 | Schwelle |
| 15 | Entnahmeöffnung |
| 16 | Standvorrichtung |
| 17 | Vorderseite |
| 17.1 | Unterkante |
| 18 | Oberseite |
| 19 | Scharnier |
| 22 | Seitenwand |
| 26 | Zugriffsöffnung |
| 27 | Aussparung |
| 28 | Endbereich |
| 29 | Schieberzunge |
| | |
| Rquer | Querrichtung |
| L1 | Öffnungslänge |
| L2 | Spatellänge |
| H1 | Öffnungshöhe |
| H2 | Spatelhöhe |

## Patentansprüche

1. Vorrichtung zur Aufbewahrung und Abgabe einheitlich geformter medizinischer Spatel (2), umfassend ein Gehäuse (1.1; 1.2) mit einem, in Form eines quaderförmigen Hohlraums ausgestalteten Lagerraum (9) und einem Boden (5), in dem die Spatel auf dem Boden (5) des Gehäuses (1.1; 1.2) stapelbar sind, wobei das Gehäuse (1.1; 1.2) an seinem Fußbereich unmittelbar oberhalb des Bodens (5) mit einer Entnahmeöffnung (15) für einen einzelnen Spatel (2) versehen ist, **dadurch gekennzeichnet, dass** die Öffnungslänge (L1) größer ist als die Spatellänge (L2), die Öffnungshöhe (H1) größer ist als die Spatelhöhe (H2) und dass der Boden (5) wenigstens eine Zugriffsöffnung (6; 26) aufweist.

2. Vorrichtung nach Anspruch 1, **dadurch gekennzeichnet, dass** die Zugriffsöffnung (6) sich im Mittelbereich des Bodens (5) befindet.

3. Vorrichtung nach Anspruch 1, **dadurch gekennzeichnet, dass** die Zugriffsöffnung (26) sich an einer Außenseite des Bodens (5) befindet.

4. Vorrichtung nach Anspruch 2 und 3, **dadurch gekennzeichnet, dass** der Boden mit zwei Zugriffsöffnungen ausgestattet ist, von denen eine (6) sich im Mittelbereich des Bodens (5) und die andere (26) sich an der Außenseite des Bodens (5) befindet.

5. Vorrichtung nach Anspruch 3 oder 4, **dadurch gekennzeichnet, dass** die seitliche Zugrifföffnung (26) sich auf der im Benutzungszustand rechten Außenseite des Gehäuses (1.2) befindet.

6. Vorrichtung nach einem der Ansprüche 1 bis 5, **dadurch gekennzeichnet, dass** die Zugriffsöffnung (6; 26) das Einführen einer Fingerspitze erlaubt.

7. Vorrichtung nach Anspruch 2 oder 4, **dadurch gekennzeichnet, dass** die im Boden-Mittelbereich befindliche Zugriffsöffnung (6) zur Entnahmeöffnung hin eine divergierende, abgerundete Kontur (7) aufweist.

8. Vorrichtung nach einem der Ansprüche 3 bis 5, **dadurch gekennzeichnet, dass** die an der Außenseite befindliche Zugriffsöffnung (26) durch eine wenigstens in der Vorderseite (17) des Gehäuses eingearbeitete Aussparung (27) vergrößert ist.

9. Vorrichtung nach einem der Ansprüche 1 bis 8, **dadurch gekennzeichnet, dass** die Rückwand (8) des Gehäuses (1.1.; 1.2) die rückseitige Fläche des Lagerraums (9) überragt.

10. Vorrichtung nach einem der Ansprüche 1 bis 9, **dadurch gekennzeichnet, dass** in dem den Lagerraum (9) überragenden Bereich der Rückwand (8) mindestens ein Loch (10) vorgesehen ist.

11. Vorrichtung nach einem der vorhergehenden Ansprüche, **dadurch gekennzeichnet, dass** wenigstens die Vorderseite (17) des Gehäuses (1.1; 1.2) wenigstens im Bereich der Entnahmeöffnung (15) durchsichtig ist.

12. Vorrichtung nach einem der vorhergehenden Ansprüche, **dadurch gekennzeichnet, dass** das Gehäuse (1.1; 1.2) an seiner Oberseite (18) oder an einer seiner Schmalseiten (3) mit einer Einfüllöffnung (12.1; 12.2; 12.3) für die Spatel (2) versehen ist.

13. Vorrichtung nach Anspruch 12, **dadurch gekennzeichnet, dass** die Einfüllöffnung (12.1; 12.2; 12.3) verschließbar ist, nämlich wahlweise mittels einer Schieberzunge (13.1) oder einer Schenkklappe (13.2).

14. Vorrichtung nach Anspruch einem der vorhergehenden Ansprüche, **dadurch gekennzeichnet, dass** bei einer Öffnung an einer Schmalseite ein Teil der Schmalseite (3) oberhalb des Bodens (5) mit einer Schwelle (14) abgedeckt ist.

15. Vorrichtung nach einem der vorhergehenden Ansprüche, **dadurch gekennzeichnet, dass** wenigstens das Gehäuse (1.1; 1.2) der Vorrichtung aus Kunststoff besteht.
